# EUROPEAN PATENT SPECIFICATION

(11) **EP 0 804 210 B1**
(45) Date of publication and mention of the grant of the patent: **27.11.2002**
(21) Application number: 94917635.8
(22) Date of filing: 16.05.1994
(51) Int. Cl.: A61K 35/14, A61K 48/00

(54) **METHOD OF IMMUNOMODULATION BY MEANS OF ADOPTIVE TRANSFER OF ANTIGEN-SPECIFIC CYTOTOXIC T-CELLS**
VERFAHREN ZUR IMMUNMODULATION DURCH ADOPTIVE ÜBERTRAGUNG VON ANTIGENSPEZIFISCHENZYTOTOXISCHEN T-ZELLEN
PROCEDE D'IMMUNOMODULATION PAR TRANSFERT ADOPTIF DE LYMPHOCYTES T CYTOTOXIQUES SPECIFIQUES D'UN ANTIGENE

(43) Date of publication of application: 05.11.1997
(73) Proprietor: Roche Diagnostics GmbH, 68305 Mannheim (DE)
(72) Inventor: BORDIGNON, Claudio, I-20129 Milano (IT); MAVILIO, Fulvio, I-20122 Milano (IT)
(86) International application number: EP9401573
(87) International publication number: WO95031208

(56) References cited:
- WO-A-92/05262
- WO-A-93/04167
- HUMAN GENE THERAPY, vol.5, no.3, 1994, NEW YORK NY, USA pages 381 - 397 H. HESLOP ET AL. 'Administration of neomycin resistance gene marked EBV-specific cytotoxic T lymphocytes to recipients of mismatched-related or phenotypically similar unrelated donor marrow grafts.'
- JOURNAL OF CELLULAR BIOCHEMISTRY, vol.0, no.16F, 1992, NEW YORK NY, USA page 47 S. FREEMAN ET AL. 'Tumor regression when a fraction of the tumor mass contains the HSV-TK gene.'
- TRENDS IN BIOTECHNOLOGY, vol.11, no.5, May 1993, CAMBRIDGE, GB pages 197 - 201 K. SIKORA 'Gene therapy for cancer.'
- PROCEEDINGS OF THE NATIONAL ACADEMY OF SCIENCES OF THE USA, vol.89, January 1992, WASHINGTON DC, USA pages 182 - 186 M. CARUSO ET AL. 'Selective killing of CD4+ cells harboring a human immunodeficiency virus-inducible suicide gene prevents viral spread in an infected cell population.'
- SCIENCE, vol.257, no.5067, 10 July 1992, WASHINGTON DC, USA pages 238 - 241 S. RIDDELL ET AL. 'Restoration of viral immunity in immunodeficient humans by the adoptive transfer of T cell clones.' cited in the application
- BLOOD, vol.83, no.7, 1 April 1994, NEW YORK NY, USA pages 1988 - 1997 F. MAVILIO ET AL. 'Peripheral blood lymphocytes as target cells of retroviral vector-mediated gene transfer.' cited in the application

## Description

The invention is concerned with a method for the production, and the use of, antigen-specific cytotoxic T-cells (CTLs), and a process for the production of a therapeutic agent using such T-cells, as well as a composition of CTLs suitable for use as a therapeutic agent.

Adoptive transfer of antigen-specific T-cells causes in animal models an efficient immunity and is, therefore, a therapeutic method for the treatment of viral infections and tumors.

In immunosuppressed patients, reactivation during chronic infections, for example by cytomegalovirus, Epstein Barr Virus or viruses from the hepatovirus groups, leads to life-threatening syndromes. This affects predominantly patients who have undergone transplantation of bone marrow or transplantation of solid organs, patients subjected to chemotherapy or radiotherapy, and HIV-infected patients.

Epstein Barr Virus (EBV) is a human herpes virus that normally replicates in epithelial cells of oropharingeal tract, and is able to immortalize B-lymphocytes in vitro. In the immunocompetent host, EBV is the causing agent of infectious mononucleosis. It is however associated with several human cancers in vivo, such as Burkitt's lymphoma, nasopharingeal carcinoma, and B-cell lymphomas in immunodeficient patients: primary immunodeficiencies, HIV-infected patients, organ transplants recipients, and patients treated with immunosuppressive drugs for autoimmune diseases (Hanto et al., 1985 *(1)*; Forman et al., 1987 *(2)*; Zutter et al., 1988 *(3)*; Kamel et al., 1993 *(4)*). The occurrence of these EBV-induced lymphoproliferations is in the majority of cases related to the degree of immunosuppression (Horowitz et al., 1990 *(23)*).

In the normal host, EBV-induced lymphoid proliferations are controlled by EBV-specific and MHC-restricted T-lymphocytes, able to be cytotoxic toward EBV-transformed cells (Royston et al., 1975 *(5)*; Rickinson et al., 1980 *(6)*), by MHC-unrestricted cytotoxic T-lymphocytes (Duncombe et al., 1992 *(7)*) and by antibodies directed toward specific viral antigens. On the contrary, in the immunodeficient patient the proliferation of these EBV-induced cells can progress without control. It is supposed that these conditions are at their beginning of polyclonal origin. However, with the persistence of the immunosuppression, clones can become neoplastic, consequently configurating the occurrence of a true EBV-induced lymphoma (Hanto et al., 1985 *(1)*).

Heslop et al. describe, in Human Gene Therapy 5 (1994) 381-397, the administration of neomycin resistance gene marked EBV specific cytotoxic T lymphocytes to recipients of mismatched-related or phenotypically similar unrelated donor marrow grafts. Such CTLs are transduced with the neomycin resistance marker gene for the purpose of tracing the infused T cells, whereby however the transduced cells were not selected on the basis of the resistance marker gene.

Many works have pointed on the fact that the removal of drug-induced immunosuppression, as in the case of solid organ recipients, could be sufficient for a spontaneous remission of this condition. If the immunosuppression persists, however, an overt neoplasm develops. Cases of EBV-induced BLPD (B-cell lymphoproliferative disorders) following bone marrow transplantation have been described (Zutter et al., 1988 *(3)*; Shapiro et al., 1988 *(8)*). The probability of developing such a proliferation is strictly related to the features of the bone marrow transplant performed. Sporadic reports of EBV-induced BLPD have been described in the setting of T-cell depleted bone marrow transplantation or in vivo use of anti-thymocyte globulin (Zutter et al., 1988 *(3)*; Shapiro et al., 1988 *(8)*). Differently than in drug-induced immunosuppression of solid organ recipients, in bone marrow transplant recipients the removal of pharmacologic immunosuppression administered for GVHD (Ferrara et al., 1991 *(31)*; Jadus et al., 1992 *(32))* or bone marrow rejection cannot be done with the objective of a fast immunologic reconstitution (Zutter et al., 1988 *(3)*; Shapiro et al., 1988 *(8)*). For this reason, the prognosis of EBV-induced BLPD in the setting of BMT has up to now been dramatically severe.

Since limited number of specific cytotoxic T-lymphocytes are required for controlling EBV-transformed B-lymphocytes in normal individuals, the administration of donor lymphocytes for the occurrence of BLPD in recipients of T-cell depleted bone marrow transplants could control this severe complication by providing to the patient donor immunity against EBV (Kernan et al., 1989 *(10)*. However, potential risks are represented by the development of a severe GVHD, that could by itself, and by the immunosuppressive drugs used for its treatment, be responsible for a relapse of EBV-induced disease.

It was shown by Riddell et al. (1992) *(22)* that by the adoptive transfer of cloned T-cells specific for CMV, antiviral immunity in immunodeficient humans could be restored. In this study, CD3⁺, CD8⁺, CD4⁻ CTL clones which are specific for a CMV antigen were generated from three CMV seropositive bone marrow donors and propagated in vitro for 5 to 12 weeks before adoptive transfer. These clones were representative of the Class I MHC-restricted immunodominant protective CTL response. During T cell transfer with these CTLs the patients received as prophylaxis for graft-versus-host disease immunosuppressive therapy with Cyclosporine A and Prednisone.

However, this method suffers from the drawback that, in order to obtain specific CTLs, individual clones have to be selected and propagated over a period of at least five to six weeks. Moreover, the CTL clones so obtained are directed in each case against one certain epitope of a virus antigen and are restricted for only one MHC class. Another drawback occurring in therapeutic use is the necessity of an additional immunosuppressive therapy. Especially in the case of patients suffering from AIDS, or patients who had to previously undergo chemotherapy or radiotherapy, such therapy is however not the treatment to be recommended.

The object of the invention is to avoid these drawbacks and provide an efficient method for the production of antigen-specific CTLs which are suitable for use, and efficient. as therapeutic agents, without requiring a long period of production or involving serious side effects.

The subject-matter of the invention is a method for the production of antigen-specific cytotoxic T-cells (CTLs), preferably CD8⁺ CTLs, by incubating a T-cell-containing cell preparation with said antigen and isolating said antigen-specific CTLs, characterized by
i) activating proliferation of CTLs in the cell preparation by incubation with the antigen;
ii) selectively transferring a vector into the proliferating CTLs, said vector containing a marker gene by means of which marked and non-marked cells can be separated; and
iii) separating the transfected antigen-specific CTLs on the basis of said marker gene.

An essential advantage of this method lies in obviating the need to select individual CTL clones in a time-consuming procedure; according to the method described here, CTLs can be obtained and separated by means of marker gene transfer followed by positive selection for a genetic marker. Furthermore, the so prepared CTL preparation contains at least two, but preferably a plurality of CTLs which present different epitopes of the antigens in the MHC complex. Here both CD4⁺ (MHC Class II restricted) and CD8⁺ (MHC Class I restricted) CTLs are obtained. In a preferred embodiment, the ratio of CD8⁺ CTLs and CD4⁺ CTLs can be controlled by interleukin-2 at predetermined concentrations, and by addition over a predetermined period. Preferably, the CTL preparation contains an excess of CD8⁺. It is also preferred to prepare, by applying suitable measures, a CD4⁻ CTL preparation (using, e.g., a cytokine additive).

As T-cell preparation there can be employed both an autologous and an allogenic preparation. Though this is not required, prior to carrying out the method according to the invention, the T-cells can be enriched or separated from other cells, and this enriched preparation can be used for the method of the invention.

As T-cells there are used preferably T-cells from an allogenic donor (of the suitable HLA type). Preferably, there is used a blood preparation which has been purified (e.g. by means of a Ficoll® gradient) so as to be free from any other blood cells. A preparation of this type is usually termed "buffy coat".

As vectors, there are preferred viral vectors which can act as a gene-delivery system in the transduction of cells. As viral vectors, there are preferred retroviruses, adenoviruses, adeno-associated viruses and herpes viruses. Retroviral vectors are the best characterized viral vectors for gene transfer and have been employed in initial gene therapy protocols (Mulligan in Nobel Symposium 80: Etiology of human diseases at the DNA level (Lindsten and Petterson, eds. 143-189, Raven Press). Adenoviral vectors may be also attractive because they are structurally stable and can be prepared at high titer (Berkner in Current Topics in Microbiology and Immunology 158 (1992), Muzyczka, N. (ed.) 36-66, Springer Verlag). Vectors derived from the non-pathogenic human adeno-associated virus (AAV) are also promising tools for human gene transfer (Muzyczka, supra, 97-129).

Since retroviral vectors infect almost exclusively dividing cells, positive selection of transduced cells allows a significant enrichment of antigen-specific lymphocytes. Therefore, retroviral vectors are preferred.

In a preferred embodiment of the invention, as an antigen there are used viral antigens, such as antigens of CMV, HBV, EBV, HIV, HSV and HCV. Depending on the basic disease, also other antigens specific for various viruses, bacteria or monocellular pathogenic organisms could be used. It is also preferred to use complete inactivated viruses, parts of viruses, isolated antigens, or preferably antigen-presenting cells as antigens. For example, patient tumor cells, infected cell lines or infected cells of the patients are useful. The use of viruses or parts thereof offers the advantage that not only one type of antigen-specific CTLs is generated but a lot of different CTLs which are directed to different antigens of the virus and/or different epitopes of the viruses. It is also preferred to use a combination of two or more virus antigens or viruses as antigens.

As the vector gene, genes coding for molecules presented at the surface of cells, such as, e.g., receptors, are preferred. In the case of blood cells, the LNGFR receptor is preferred (Bordignon et al., 1994 *(18)*)*.*

Immunomodulation means transient transfer of antigen-specific T-cells for the stimulation of the immune response of the patient against tumor cells or infected cells.

These gene-modified CTLs contain preferably, in addition, a suicide gene (which expresses after induction a substance which causes directly or by mediators the death of the infected cell; e.g. WO 92/08796) for in vivo specific elimination of these cells after successful treatment. For this purpose, there is preferably applied the thymidine-kinase gene, which confers to the transduced CTLs in vivo sensitivity to the drug Ganciclovir for in vivo specific elimination of cells potentially responsible for GVHD. If, for example, the patients develop signs of acute GVHD with increasing liver function enzymes and a positive skin biopsy, it is preferred to administer i.v. two doses of about 10 mg/kg of the drug Gancyclovir. This results in a reduction of marked lymphocytes only to a minor extent.

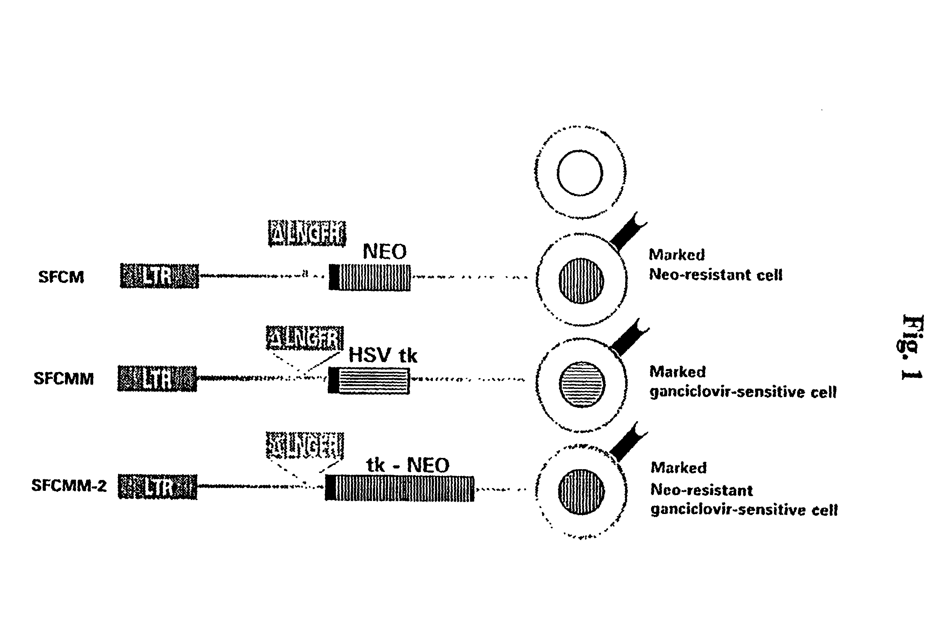

Preferred types of gene-modified CTLs are shown in Fig. 1.

The diphtheria toxin gene is also preferred as a suicide gene, which is described in WO 92/05262 *(36)*. For the in vivo specific elimination of the CTLs after a GVHD it is also possible to induce a cell apoptosis. It is thus preferred to use a modified FAS-receptor and a dose of a related ligand.

The strategy according to the invention may find a wide application in adoptive immunotherapy approaches where a very large number of donor lymphocytes are utilized, for example in controlling relapsing leukemic cells after allo-BMT (Kolb et al., 1990 *(25)*; Riddel et al., 1992 *(26)*; Cullis et al., 1992 *(27)*; Klingemann et al., 1991 *(28)*; Helg et al., 1993 *(29)*; Bar et al., 1993 *(30)*).

An additional advantage of the positive selection of antigen-specific cells through vector infection and positive sorting of vector-gene-expressing cells is the production of a population of donor lymphocytes containing a higher frequency of tumor-specific cells, while at the same time reducing the number of alloreactive cells potentially causing GVHD. It is noteworthy to consider that this technique may find application in other forms of adoptive immunotherapy of tumors expressing known tumor associated antigens (Traversari et al., 1992 *(33)*; van der Bruggen et al., 1991 *(34)*).

In a preferred embodiment of the invention, IL-2 is added at a concentration of from 10 to 50 units/10⁵ cells, preferably at about 10 to 25 units/about 10⁵ cells.

In a further embodiment of the invention, it is preferred to add. in addition, IL-4 at a concentration of about 5 units/10⁵ cells.

Surprisingly it was found that for the production of vector-transduced antigen-specific CTLs it is preferred to cultivate in the presence of IL-2 the lymphocytes after activation of the proliferation of the CTLs with the antigen for 2 to 30 days, preferably 2 to 10 days, before infecting the proliferating CTLs with the vector. After the infection with said vector, cells will be cultivated also in the presence of IL-2 for another period of 2 to 4 days. Then the selection on the basis of the marker gene is carried out and the (preferably CD8⁺) CTLs are isolated.

The infection of T-cells with viral vectors can be accomplished by co-cultivation of vector virus-producing cells with said T-cells with supernatant from vector virus-producing cells or by infection with purified viruses.

It is also preferred to use tumor cell-specific antigens, tumor cells or cells presenting such antigens, for the activation of T-cells. An example hereof is the MAGE-antigen which is specific for a part of the malignant melanomas or a part of the mastocarcinomas (van der Bruggen et al., 1991 *(34)*).

The invention is further illustrated by the figure and the examples set forth below.

### REFERENCES

1 Hanto DW, Frizzera G, Gail Peczalska KJ, Simmons RL: Epstein Barr virus, immunodeficiency, and B cell lymphoproliferation. Transplantation 1985; 39: 461-472.
2 Forman J.S., Sullivan J.L., Wright C., Ratech H., Racklin B., Blume K.G.: Epstein-Barr-Virus related malignant B cell lymphoplasmacytic lymphoma following allogeneic bone marrow transplantation for aplastic anemia. Transplantation 1987; 44: 244-249
3 Zutter MM, Martin PJ, Sale GE, Shulman HM, Fischer L, Thomas ED, Durnam DM: Epstein-Barr Virus Lymphoproliferation after bone marrow transplantation. Blood 1988;72:520-529.
4 Kamel O.W., Van de Rijn M., Weiss L.M., et al: Reversible lymphomas associated with Epstein Barr virus occurring during methotrexate therapy for rheumatoid arthritis and dermatomyositis. N. E.J.Med. 1993; 328: 1317-1321
5 Royston I, et al: Cell mediated immunity to EBV transformed lymphoblastoid cells in acute infectious mononucleosis. N.E.J.Med. 1975; 293: 1159.
6 Rickinson AB, Wallace L.E., Epstein M.A: HLA restricted T cell recognition of EBV infected B cells. Nature 1980; 283: 865
7 Duncombe AS, Grundy JE, Oblakowsky P, Prentice HG, Gottlieb DJ, Roy DM et al: Bone marrow transplant recipients have defective MHC-unrestricted cytotoxic responses against cytomegalovirus in comparison with Epstein-Barr virus: the importance of target cell expression of lymphocyte function associated antigen 1 (LFA-1). Blood 1992; 79: 3059-3066.
8 Shapiro RS, McClain K, Frizzera G. et al: Epstein-Barr Virus Associated B cell Iymphoproliferative disorders following bone marrow transplantation. Blood 1988;71:1234-1243.
9 WO 89/07150
10 Kernan N.A., Bordignon C., Collins N.H., Castro-Malaspina H., Cunningham I., Brochstein J., Shank B., Flomenberg N., Dupont B., & R.J. O'Reilly: Bone marrow failure in HLA-identical T-cell depleted allogeneic transplants for leukemia: I. Clinical aspects. Blood 74:2227-2236 (1989).
11 Papadopoulos E.B., Ladanyi M., Emanuel D., Mackinnon 5., Rosenfield N.S., Boulad F., Carabasi M.H., Castro-Malaspina H., Childs B.H., Gillio A.P., Small T.N., Young J.W., Kernan N.A., O'Reilly R.J.: Infusions of donor leukocytes as treatment of Epstein-Barr virus associated lymphoproliferative disorders complicating allogeneic marrow transplantation. NEJM 1994 in press.
12 Reisner Y,Reisner Y, Kapoor N, et al ... O'Reilly R: Transplantation for acute leukemia with HLA-A and B non identical parental marrow cells fractionated with soybean agglutinin and sheep red blood cells. Lancet 1981 2: 327
13 Bordignon C., Kernan N.A., Keever C.A., Cartagena T., Benazzi E., Burns J., Flomenberg N., Dupont B., & R.J. O'Reilly: Bone marrow failure in HLA-identical T-cell depleted allogeneic transplants for leukemia: II. Experimental correlates. Blood 74:2237-2241 (1989).
14 Clinical Protocol
15 Moolten F.L, Wells J.M., Heyman R.A., Evans R.M. Lymphoma regression induced by gancyclovir in mice bearing a herpes thymidine-kinase transgene. Hum. Gene Ther. 1990, 1:125-134.
16 Wu T.C., Mann R.B., Epstein J.I., Mac Mahon E., Lee W.A., Charache P., Hayward S.D., Curman R.J., Hayward G.S., Ambinder R.F. Abundant expression of EBER 1 small nuclear RNA in nasopharyngeal carcinoma. Am. J. Pathol. 1991; 138:1461-1469.
17 McCann S.R. Lawler M. Mixed chimaerism; detection and significance following BMT. Bone Marrow Transpl. 1993; 11:91-94.
18 Mavilio F., Ferrari G., Rossini S., Bonini C., Casorati G., & C. Bordignon: Retroviral vector mediated gene transfer into human peripheral blood lymphocytes for human gene therapy. Blood 83(1994) 1988-1997
19 Langhorne J., and Fischer-Lindahl K.: Limiting dilution analysis of precursors of cytotoxic T-lymphocytes. J. Immunol. Methods 11:221 (1981).
20 Bourgault I., Gomez A., Gomard E., Levi J.P.: Limiting dilution analysis of the HLA restriciton of anti-Epstein-Barr virus specific cytolytic T-lymphocytes. Clin. exp. Immunol. 1991,84: 501-507.
21 Taswell C.: Limiting dilution assay for the determnination of immunocompetent cell frequencies. I Data analysis. J. Immunol. 126:1614 (1981).
22 Ridell et al., Science 1992, 257:238-241
23 Horowitz M.M., Gale R.P., Sondel B.M., Goldman J.M., Kersey J., Kolbe H.J., Rimm A.A., Ringden O, Rozman C., Speck B., Truitt R.L., Zwaan F.E., Bortin M.M. Graft-versus-leukemia reactions after bone marrow transplantation. Blood 1990, 75:555-562.
24 Markowitz D., et al., J. Virol. 1988, 62:1120-1124
25 Kolb HJ, Mittermuller J, Clemm Ch, Holler E, Ledderose G, Brehm G, Heim M, Wilmanns W: Donor leukocyte transfusions for treatment of recurrent chronic myelogenous leukemia in marrow transplant patients. Blood 1990, 76: 2462-2465.
26 Riddel S.R., Watanabe K.S., Goodrich J.M., Li C.R., Agha M.E., Greenberg P.D. Restoration of viral immunity in immunodeficient humans by the adoptive transfer of T-cell clones. Science 1992, 257:238-241.
27 Cullis JO, Jiang YZ, Schwarer AP, Hughes TP, Barrett AJ, Goldman JM: Donor leukocyte infusions for chronic myeloid leukemia in relapse after allogeneic bone marrow transplantation Blood 1992, 79: 1379-1380
28 Klingemann HG, Phillips GL: Immunotherapy after bone marrow transplantation Bone Marrow Transplantation 1991, 8: 73-81.
29 Helg C, Roux E, Beris P, Cabrol C, Wacher P, Darbellay R, Wyss M, Jeannet M, Chapuis B, Roosnek E.: Adoptive immunotherapy for rccurrent CML after BMT. Bone Marrow Transplantation 1993, 12: 125-129.
30 Bar BMAM, Schattenberg A, Mensink EJBM, Geurt Van Kessel A, Smetsers TFCM, Knops GHJN, Linders EHP, De Witte T: Donor leukocyte infusions for chronic myeloid leukemia relapsed after allogeneic bone marrow transplantation Journal of Clinical Oncology 1993,3:513-519.
31 Ferrara J.L.M., Deeg H.J. Graft-versus-host disease N. Engl. J. Med. 1991,324:667-674.
32 Jadus MR, Wepsic HT: The role of cytokines in graft versus host reactions and disease Bone marrow transplantation 1992, 10: 1-14.
33 TRAVERSARI C, VAN DER BRUGGEN P, VAN DEN EYNDE B, HAINAUT P, LEMOINE C, OHOTA N, OLD L, BOON T: Transfection of the gene coding for the expression of a human melanoma antigen recognized by autologous cytolytic T lymphocytes. Immunogenetics 1992, 35:145-148.
34 VAN DER BRUGGEN P, TRAVERSARI C, CHOMEZ P, LURQUIN C, DE PLAEN E, VAN DEN EYNDE B, KNUTH A, BOON T: A gene encoding an antigen recognized by cytolytic T lymphocytes on a human melanoma. Science 1991, 254:1643-1647.
35 Markowitz D., et al., Virology 1988, 167:400-406
36 WO 92/05262

### Example 1

### Mixed lymphocyte tumor culture (MLTC) for production of vector-transduced antigen-specific CTLs

On day 0: autologous PBLs were resuspended in Iscove's medium supplemented with L-arginine (0.55 mM), L-asparagine (0.24 mM), L-glutamine (1.5 mM) in the presence of 10 % human serum (HS). HS was pooled A B and O Serum from healthy donors, decomplemented (56°C, 30 min), and sterilized.

One million responder PBL were mixed in multi-dish 24 wells with 10⁵ stimulator autologous tumor cells expressing EBV antigens, irradiated (10,000 rads are enough for the tumor cell lines used till now) in a final volume of 2 ml of the above-described medium, in the presence of 20 ng/ml r-hu-IL4 (5 U). Higher concentrations induce IL-2 production and block the activity of exogenous IL-2, thus inhibiting cytotoxicity.

On day 3: r-hu-IL2 was added at the final concentration of 10 - 25 units/ml and infectious viruses were added. On day 7: responder lymphocytes were collected, centrifuged, counted and resuspended in fresh medium. Lymphocytes 3 - 5 x 10⁵ were restimulated in 24-well plates with 10⁵ irradiated tumor cells in 2 ml of the same medium containing 10 - 25 units/ml of IL-2 and 20 ng/ml of IL-4. On day 14: responder lymphocytes were restimulated as at day 7, or cloned by limiting dilution. MLTC responder lymphocytes were cloned by limiting dilution in 96-well microtiter plates (round-bottomed wells). 10, 3, 1 and 0.3 responder cells were seeded in 100 µl of medium containing 50 units/ml of IL-2, 3000 irradiated (10,000 rads) tumor cells and 5 x 10⁴ irradiated (6000 rads) allogeneic PBLs as feeder cells. On day 21: clones were restimulated by adding in 100 µl of medium containing 50 units/ml of IL-2, 3000 irradiated (10,000 rads) tumor cells and 5 x 10⁴ irradiated (6000 rads) allogeneic PBLs as feeder cells. The frequency of EBV-specific donor lymphocytes, after exposure to irradiated EBV-infected autologous B-cells, and positive sorting of vector transduced Iymphoctes increased of over 100 folds.

This time kinetic is providing the highest efficiency of production of gene-transduced, highly cytotoxic CTLs.

### Example 2

### Preparation of the infectious viruses

Vector DNAs were converted to corresponding viruses by a transinfection protocol. Briefly, vector DNA was transfected *into* the psi2 ecotropic packaging line (Mann et al., Cell 33 (1983) 153) by standard calcium phosphate co-precipiptation. 48 hrs after transfection psi2 supernatants were harvested and used to infect the amphotropic packaging cell line PA317 (Miller et al., Mol. Cell Biol. 6 (1986) 2895) for 16 hrs in the presence of 8 µg/ml polybrene. Infected PA317 cells were selected in DMEM (Gibco, Grand Island, New York), supplemented with 10 % FCS (Hyclone, Logan, UT) and containing 0.8 mg/ml G418 (Gibco), and then used to generate helper-free virus-containing supernatants with titers ranging from 10⁴ : 10⁵ CFU/ml. All vectors contained a gene neoR gene coding for neomycinphosphotransferase that confers in vitro resistance to the neomycin analogue G418.

Instead of psi2 and PA317, also packaging cell lines of higher safety standard can be used (e.g. E86 and AM12 (Markowitz et al., 1988 *(35)*; Markowitz et al., 1988 *(24)*; WO 89/07150 *(9)*).

### Infection of antigen-specific CTLs

Viral infection was carried out by exposure of stimulated CTLs (according to Example 1) to a cell-free viral stock for 6 hrs in the presence of polybrene (8 µg/ml). 48 hrs after infection PBLs were selected in RPMI1640, supplemented with 2 mmol/l L-glutamine, 1 % non-essential amino acids, 1% Na pyruvate, 5 % human serum (HS), and 100 U/ml r-hu-IL2 (complete medium) containing 0.4 mg/ml G418. Cell density was maintained constant (5 x 10⁵ cells/ml) during two weeks of G 148 selection. Retroviral transducted human T-lymphocytes were also cloned in Terasaki plates at different cell concentrations (1 x 10³ cells/well) in complete medium containing 0.4 mg/ml G418 in the presence of irradiated human CTLs as feeder cells.

In order to improve the retroviral infection efficiency, human CTLs were co-cultivated with virus-producing cells for 48 to 72 hrs in complete medium. Cocultivation was also carried out in transwell plates (Costar, Cambridge, MA) to prevent cell-to-cell contact. 3 x 10⁵ producer cells were seeded in the cluster plate wells of 6-well dishes and incubated at 37°C overnight. 5 x 10⁵ stimulated CTLs were added into the transwells and grown for 48 to 72 hrs in the presence of 8 µg/ml polybrene.

Retroviral transduced cells were analyzed by flow cytometry for receptor expression and expanded for further analyses.

### Example 3

### Case Report

A 29 year-old woman with grade G lymphoma, in second remission after 1 year of treatment with conventional chemotherapy, received a T-cell depleted bone marrow transplantation from her HLA-identical and MLC-compatible brother The pretransplant conditioning was performed accordingly with Kernan et al. *(11)* with modifications, and included TBI (1320 cGy in 11 fractions over four days), Cyclofosfamide (60 mg/Kg/die in each of two successive days), VP16 (375 mg/mq/die in each of two successive days), and rabbit Antithymocyte Globulin (5 mg/Kg/die in each of four successive days) administered over four days pretransplant. T-cell depletion was performed by soybean lectin agglutination and E-rosetting *(12).* In order to prevent graft rejection *(13)*, the patient received prednisone (1 mg/kg/day) for the first 60 days post-transplantation *(12).* The patient was discharged from the hospital on the day 40 post-transplant, with a documented engraftment, in good conditions.

Between day 55 and 60, the patient developed a right laterocervical mass, accompanied by high fever, sharp fall in peripheral blood counts, and increase of AST, ALT, LDH and alkaline phosphatase (see results). An ecotomographic analysis of the neck mass showed multiple packed lymphonodes of about 2 cm each. At a chest CT-scan numerous smaller right paratracheal adenopathies were observed. A laterocervical lymphonode biopsy was performed and confirmed the presence of a diffuse large cell lymphoma, associated with areas of necrosis. The in situ hybridization for EBV showed the presence of EBV RNA in the nuclei of the neoplastic cells. A myeloaspirate and a bone marrow biopsy showed a myelodisplastic pattern and an overt infiltration of the bone marrow by lymphoid paratrabecolar nodules. On this basis, the diagnosis of EBV-induced secondary lymphoproliferation was made. The possibility of an adoptive immunotherapy by the administration of donor lymphocytes that could control this severe complication by providing to the patient donor immunity against EBV was considered *(10)*.

A clinical protocol for utilization of gene-modified donor lymphocytes in the contest of allogeneic transplantation was approved by the Ethical Committee of Istituto Scientifico San Raffaele in accordance with the guidelines of the National (Italian) Committee for Biosafety on May 15, 1993 *(14)*. Accordingly, with this protocol, donor lymphocytes are transduced in vitro by a retroviral vector for transfer and expression of two genes: 1 - a modified (non-functional) form of the low affinity receptor for the nerve growth factor gene (ΔLNGFR), for in vitro selection of transduced cells and for in vivo follow-up of the infused donor lymphocytes; 2 - the thymidine-kinase gene that confers to the transduced PBL in vivo sensitivity to the drug ganciclovir *(15)*, for in vivo modulation of donor-anti tumor response, and for in vivo specific elimination of cells potentially responsible for GVHD.

After diagnosis, the patient's informed consent was obtained, and she received 2 x 10⁶/Kg CD3⁺ lymphocytes obtained from her donor brother. in two subsequent infusions. The detailed description of the clinical impact of infusion of donor lymphocytes is reported in the Results section. In a brief summary, the patient cleared the clinical signs of EBV lymphoma in two weeks from the first infusion of donor lymphocytes. Due to the development of grade II G she received ganciclovir treatment that resulted in complete elimination of marked PBLs from the peripheral blood, was discharged from the hospital on day 116 from BMT, with no signs of EBV lymphoproliferation, GvHD, nor underlying disease. The frequency of anti-EBV lymphocytes in her blood was in the range of 1/3000. This compares to normal individuals, including her transplant donor who has a frequency of 1/1100.

### Clinical impact of the infusion of donor lymphocytes

In the two weeks following the administration of vector-transduced donor cells. all clinical symptoms associated with EBV-induced B-cell proliferation regressed, with full hematopoietic recovery. During this time, marked donor cells increased progressively in the patient peripheral blood up to 13.4 % of total mononuclear cells, as detected by FACS analysis of LNGFR-expressing cells. As indicated in Figure 3, at the time of regression of clinical symptoms, a sharp increase in PBL counts was observed. Circulating transduced donor lymphocytes were almost exclusively CD3⁺/CD8⁺ lymphocytes (>90 % of total mononuclear cells from day +10 to day +15), with high proliferation index. More than 1/10 cells recognized EBV-infected autologous B-cells in vitro.

### Immunomodulation of donor vs host alloreactivity in vector-transduced lymphocytes

Approximately four weeks after infusion of gene-modified lymphocytes, the patient progressively developed signs of acute GVHD, with increasing liver function enzymes, and a positive skin biopsy. The i.v. administration of two doses of 10 mg/kg of the drug ganciclovir resulted in reduction of marked lymphocytes to 3.1 %, with disappearance of clinical signs of skin GVHD and reduction of over 50 % of all altered liver function enzymes. This patient was discharged six weeks after treatment with no apparent signs of disease.

### Methods

In situ hybridization for EBV genome: In situ hybridization for the detection of EBV was performed using deoxyribo-oligonucleotides of 30-mers complementary to the two nuclear EBER RNAs encoded by Epstein-Barr Virus (Dako, Glostrup, Denmark) *(16)*.

Analysis of the chimerism in the patient BM, PBLS, and the EBV-lymphoma infiltrated lymphonode was performed by PCR polymorphism of ApoB, ApoC, and YNZ22 *(17)*.

Gene transfer into peripheral blood lymphocytes is accomplished in accordance with Example 1 and Reference *(18).*

Determination of antigen-specific lymphocytes precursors frequencies: The frequency of EBV-specific T-cells and allospecific T-cells was performed by limiting dilution as described in *(19)* with minor modifications. Briefly, PBMC were co-cultured in limiting numbers in round-bottom microtiter plates with 2 x 10⁴ irradiated (5000 rads) autologous EBV-transformed B-cells, or 4 x 10⁴ irradiated (3000 rads) allogenic PBMC as stimulators, and 10³ irradiated (3000 rads) autologous PBMC as feeders in 200 µl of supplemented RPMI 1640 (Gibco, containing 5 % autologous serum). 24 concentrations were set-up for each responder dilution. 20 units of hr-IL-2 were added on day 6. A chromium release assay was performed on days 8 and 12 using autologous EBV-B cells or allogeneic PBMC as targets. The proliferation assay was performed, after identical culture conditions, on day 8 by an incorporation of 3H-thymidine (Amershan) for 6 hrs. Precursor frequency was calculated by Poisson distribution relationship between the responding cell number and log of the percentage of non-responding cultures as described *(20, 21)*.

Cell surface phenotyping: Cell surface expression of LNGFR was monitored by flow cytometry utilizing the murine anti-human LNGFR monoclonal antibody 20.4 (ATCC) with an indirect fluorescence labelling method *(18)*. Cell surface phenotype of T-lymphocytic lines and clones was determined by flow cytometry using PE-conjugated anti-human-CD4 (T4), CD8 (T8), CD5, B4, CD25R, Leu7, CD34 monoclonal antibodies (MoAb) (Coulter Immunology, Hialeah. FL). Briefly, 5 x 10⁵ cells were stained with 100 ml of diluted antibodies at 4°C for 30 min., washed twice in medium without FCS and resuspended in 0.5 ml of PBS for FACS analysis or in 100 ml of diluted FITC-conjugated secondary antibody. Double-staining analysis was performed by sequential incubation of FITC- and PE-conjugated antibodies.

## Claims

1. Method for the production of antigen-specific cytotoxic T-cells (CTLs) by incubating a T-cell-containing cell preparation with said antigen and isolating said antigen-specific CTLs, **characterized by**
i) activating proliferation of CTLs in the cell preparation by incubation with the antigen;
ii) selectively transferring a vector into the proliferating CTLs, said vector containing a marker gene by means of which marked and non-marked cells can be separated; and
iii) separating the transfected antigen-specific CTLs on the basis of said marker gene.

2. The method according to claim 1, **characterized by** using a retrovirus as said vector.

3. The method according to claim 1 or 2, **characterized by** using, as the marker gene, the low affinity NGF receptor.

4. The method according to claims 1 to 3, **characterized in that** the vector contains in addition a suicide gene.

5. The method according to claim 4, **characterized by** using, as a suicide gene, the TK gene of HSV.

6. The method according to claims 1 to 5, **characterized by** using, as said antigen, inactivated viruses or parts thereof, isolated antigens or antigen-presenting cells.

7. The method according to claim 6, **characterized by** using, as viruses, CMV, HBV, EBV, HIV, HSV and HCV.

8. The method according to claims 1 to 7, **characterized by** cultivating the activated CTLs in the presence of IL-2 for a time period of 2 to 30 days, infecting the cells with said vector, cultivating the cells in the presence of IL-2 for another period of 2 to 4 days, and selecting CTLs on the basis of said marker gene.

9. The method according to claim 8, wherein CD8⁺ CTLs are selected.

## Patentansprüche

1. Verfahren zur Herstellung von Antigen-spezifischen cytotoxischen T-Zellen (CTLs) durch Inkubieren einer T-Zellen-enthaltenden Zellpräparation mit dem Antigen und isolieren der Antigen-spezifischen CTLs,
**dadurch gekennzeichnet, dass**
i) die Proliferation der CTLs in der Zellpräparation durch Inkubation mit dem Antigen aktiviert wird;
ii) ein Vektor selektiv in die proliferierenden CTLs transferiert wird, wobei der Vektor ein Markergen enthält, durch das markierte und nichtmarkierte Zellen voneinander getrennt werden können; und
iii) die transfizierten Antigen-spezifischen CTLs aufgrund des Markergens abgetrennt werden.

2. Verfahren nach Anspruch 1,
**dadurch gekennzeichnet, dass**
als Vektor ein Retrovirus verwendet wird.

3. Verfahren nach Anspruch 1 oder 2,
**dadurch gekennzeichnet, dass**
als Markergen der niedrig affine NGF-Rezeptor verwendet wird.

4. Verfahren nach einem der Ansprüche 1 bis 3,
**dadurch gekennzeichnet, dass**
der Vektor zusätzlich ein Suizidgen enthält.

5. Verfahren nach Anspruch 4,
**dadurch gekennzeichnet, dass**
als Suizidgen das TK-Gen von HSV verwendet wird.

6. Verfahren nach einem der Ansprüche 1 bis 5,
**dadurch gekennzeichnet, dass**
als Antigen inaktivierte Viren oder Teile davon, isolierte Antigene oder Antigen-präsentierende Zellen verwendet werden.

7. Verfahren nach Anspruch 6,
**dadurch gekennzeichnet, dass**
als Viren CMV, HBV, EBV, HIV, HSV und HCV verwendet werden.

8. Verfahren nach einem der Ansprüche 1 bis 7,
**dadurch gekennzeichnet, dass**
die aktivierten CTLs in Gegenwart von IL-2 für eine Zeitdauer von 2 bis 30 Tagen kultiviert werden, die Zellen mit dem Vektor infiziert werden, die Zellen in Gegenwart von IL-2 für eine weitere Zeitdauer von 2 bis 4 Tagen kultiviert werden und die CTLs aufgrund des Markergens selektiert werden.

9. Verfahren nach Anspruch 8,
**dadurch gekennzeichnet, dass**
CD8⁺-CTLs selektiert werden.

## Revendications

1. Procédé pour la production de cellules T cytotoxiques spécifiques à l'antigène (CTL) par incubation d'une préparation cellulaire contenant des cellules T avec ledit antigène, et isolement desdites CTL spécifiques à l'antigène, **caractérisé par** les étapes consistant à :
i) activer la prolifération de CTL dans une préparation cellulaire par incubation avec un antigène ;
ii) transférer sélectivement un vecteur dans les CTL proliférant, ledit vecteur contenant un gène marqueur, au moyen duquel les cellules marquées et non marquées peuvent être séparées ; et
iii) séparer les CTL spécifiques à l'antigène transfectées sur la base dudit gène marqueur.

2. Procédé selon la revendication 1, **caractérisé par** l'utilisation d'un rétrovirus en tant que dit vecteur.

3. Procédé selon la revendication 1 ou 2, **caractérisé par** l'utilisation, en tant que gène marqueur, du récepteur au NGF de faible affinité.

4. Procédé selon les revendications 1 à 3, **caractérisé en ce que** le vecteur contient en outre, un gène suicide.

5. Procédé selon la revendication 4, **caractérisé par** l'utilisation, en tant que gène suicide, du gène TK du HSV.

6. Procédé selon les revendications 1 à 5, **caractérisé par** l'utilisation, en tant que dit antigène, de virus inactivés ou de parties de ceux-ci, d'antigènes isolés ou de cellules présentatrices de l'antigène.

7. Procédé selon la revendication 6, **caractérisé par** l'utilisation, en tant que virus de CMV, HBV, EBV, VIH, HSV et HCV.

8. Procédé selon les revendications 1 à 7, **caractérisé par** la culture de CTL activées en présence d'IL-2 pendant une période de temps de 2 à 30 jours, l'infection des cellules avec ledit vecteur, la culture des cellules en présence d'IL-2 pendant une autre période de 2 à 4 jours, et la sélection des CTL sur la base dudit gène marqueur.

9. Procédé selon la revendication 8, dans lequel les CTL CD8⁺ sont sélectionnées.
